# EUROPEAN PATENT APPLICATION

(11) **EP 4 353 292 A1**
(43) Date of publication of application: **17.04.2024**
(21) Application number: 23202472.9
(22) Date of filing: 09.10.2023
(51) Int. Cl.: A61M 25/00, A61B 5/00, H05K 1/11

(54) **TAPE ASSEMBLY FOR A SMART CATHETER**

(30) Priority: 11.10.2022 US 202263414938 P; 24.02.2023 US 202363448005 P; 03.10.2023 US 202318479930
(71) Applicant: Freudenberg Medical, LLC, Beverly, MA 01915 (US)
(72) Inventor: TIMOTHY, Zeis, Charlestown, IN 47111 (US)
(74) Representative: Manitz Finsterwald Patent- und Rechtsanwaltspartnerschaft mbB

(57) **Abstract**

A tape assembly (10) for transferring a circuit onto a catheter shaft includes a removable transfer media layer (12) and a circuit assembly (17) disposed in releasably bonded relationship with the transfer media layer. The circuit assembly includes a conductive circuit layer (18) having at least one circuit (20), and a dielectric layer (26) disposed in overlaying relationship with the conductive circuit layer. The circuit assembly can also include an adhesive layer (28) disposed in overlaying relationship with the dielectric layer and a seal layer (29) disposed in sandwiched relationship between the transfer media layer and the conductive circuit layer. The transfer media layer provides support for the circuit assembly during transfer to the catheter shaft, and is removable from the circuit assembly after application to the catheter shaft to provide a reduced profile for the resultant smart catheter shaft.

## Description

### CROSS REFERENCE TO RELATED APPLICATIONS

The subject application is related to U.S. Provisional Patent Application Serial No. 63/414,938 filed on October 11, 2022 and U.S. Provisional Patent Application Serial No. 63/448,005 filed on February 24, 2023.

### BACKGROUND OF THE INVENTION

### 1. Field of the Invention

The present disclosure relates generally to medical devices and procedures. In particular, the present disclosure relates to circuits for a smart catheter.

### 2. Description of the Prior Art

This section provides background information related to the present disclosure which is not necessarily prior art.

A catheter is a medical instrument for use in accessing an interior of a patient's body with a distal tip during a medical procedure. The catheter can include at least one working component, such as a fluid channel, a working channel, and/or an electronics cable, which extend along the catheter and terminate at or adjacent the distal tip. There is a growing trend in the industry to also integrate circuits into the catheter shaft, thereby providing intelligence to the catheter (i.e., a "smart catheter"). Smart catheters need to articulate and steer the distal tip through the body to reach their target destination, with tight bends being required. The catheter shaft will also bend as it pushes through the tortious path of the human body, which causes compression and expansion points on the catheter shaft. The more the catheter shaft bends, the greater the effect.

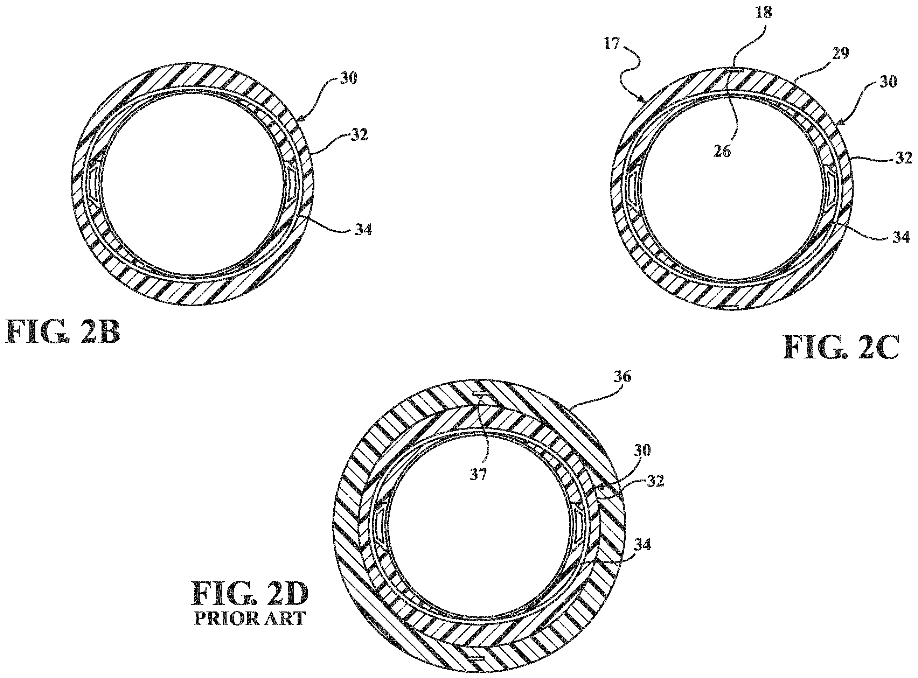

The prior art circuits are commonly integrated into the catheter shaft by flowing a flexible circuit **37** embedded in a stretchable thermoplastic polymer encasement *(See* e.g., element **36** in Figure 2D) or a less stretchable polyamide film, each of which functions as the substrate for the circuit. This substrate is required and must be present to provide structural support for the small fragile electrical pathways of the prior art circuit **37.** However, the presence of this structural support for the circuit has significant drawbacks. For example, because polyimide film does not have sufficient flexibility to accommodate the required compression and expansion encountered in a catheter shaft application, use of polyimide film as the support is limited on how and at what lengths it can be applied. On the other hand, use of the stretchable thermoplastic polymer adds significant thickness to the catheter shaft. More specifically, since stretchable materials are less rigid, they are less suitable as a structure, and more thickness must be added to compensate for this drawback. With reference to Figure 2D, in the case of a smart catheter application, the added thermoplastic polymer encasement **36** (a 0.20mm thickness being common) required to embed the circuit **37** can increase a diameter of the catheter shaft by as much as twenty times the thickness of the circuit itself (which may only be 0.01mm), which is functionally detrimental to the final diameter of the catheter product. In addition, because the prior art circuit **37** is embedded in the polymer encasement **36,** when the polymer melts during the reflow process, the circuits begin to move around the liquefying polymer, creating potential for the circuits to short out on one another or on the braid layer. In summary, in these prior art cases, the substrate must be strong enough to support the circuit structure, and after the circuit is transferred, the polymer encasement **36** support structure (e.g., the polyimide film or the thermoplastic polymer) becomes part of the catheter structure itself. This negatively impacts the size and flexibility of the resultant smart catheter. Accordingly, there remains a continuing need for improved ways of incorporating a circuit into the catheter shaft during manufacture of the smart catheter.

### SUMMARY OF THE INVENTION

This section provides a general summary of the disclosure and is not intended to be a comprehensive disclosure of its full scope, aspects, objectives, and/or all of its features.

A tape assembly for transferring at least one circuit onto a catheter shaft includes a removable transfer media layer extending between a first end and a second end. A circuit assembly is disposed in overlaying and releasably bonded relationship with the removable transfer media layer between the first and second ends, and includes a conductive circuit layer having at least one circuit. The removable transfer media layer provides temporary structural support for the circuit assembly until the circuit assembly is transferred to become part of the catheter shaft. Once the transfer is complete, the removable transfer media layer is removed and discarded, leaving only the circuit assembly behind, in secured relationship with the catheter shaft.

Relatedly, a method of manufacturing a smart catheter includes providing a catheter shaft, and providing a tape assembly including a removable transfer media layer and a circuit assembly having a conductive circuit layer. The method proceeds by applying the tape assembly to the catheter shaft to dispose the circuit assembly in overlaying relationship with the catheter shaft, and then removing the removable transfer media layer from the circuit assembly to leave the circuit assembly applied to the catheter shaft.

The tape assembly provides a means of manufacturing a better, more reliable smart catheter shaft. More specifically, the transfer of the circuit assembly directly onto the catheter shaft, without the need of including a support structure (e.g., a thermoplastic polymer encasement **36**) as part of the permanent structure of the catheter shaft, keeps the catheter's profile (i.e., diameter) at a minimum *(See,* e.g., a comparison of Figure 2B illustrating the catheter shaft prior to transfer of the circuit assembly to Figure 2C illustrating the catheter shaft with the transferred circuit assembly after removal of the transfer media layer) while allowing for the best flexibility possible. A lower profile also helps to keep the circuit positionally stable with minimal chance of moving, which could cause shorts and breaks. For comparison, if the circuit present in the conductive circuit layer was encased in a structural thermoplastic encasement prior to the transfer taking place, as required by the prior art, the thermoplastic encasing the circuit would need to be heated to a liquid state during the transfer to get the thermoplastic and circuit to stick to the catheter shaft, during which time the circuit could shift and move in the melt.

### BRIEF DESCRIPTION OF THE DRAWINGS

Other aspects and advantages of the present disclosure will be readily appreciated, as the same becomes better understood by reference to the following detailed description when considered in connection with the accompanying drawings wherein:
Figure 1A is a side view of a tape assembly constructed in accordance with a first embodiment and illustrating a removable transfer media layer and a circuit assembly disposed in removably bonded relationship with the removable transfer media layer, the circuit assembly being multi-layered and including a conductive circuit layer, a dielectric layer, and an adhesive layer;
Figure 1B is a top view of the tape assembly to more clearly illustrate the conductive circuit layer of the circuit assembly;
Figure 1C is a side view of the tape assembly constructed in accordance with a second embodiment and illustrating the circuit assembly additionally including a seal layer disposed in sandwiched relationship between the transferable media layer and the conductive circuit layer;
Figure 2A is a perspective view of a catheter shaft illustrating the tape assembly applied to the catheter shaft after which the removable transfer media layer is removed from the circuit assembly containing the conductive circuit layer, the dielectric coating, the adhesive layer (and the seal layer, if present) to transfer the circuit assembly to the catheter shaft;
Figure 2B is a cross-sectional view of the catheter shaft prior to application of the circuit assembly;
Figure 2C is a cross-sectional view of the catheter shaft after the circuit assembly is transferred to the catheter shaft and the removable transfer media layer is removed to illustrate the seal layer overlaying and protecting the conductive circuit layer from an environment of the catheter shaft and the dielectric coating protecting the conductive circuit layer from an outer surface and/or a braid layer on the catheter shaft;
Figure 2D is a cross-sectional view of the catheter shaft illustrating a prior art approach of embedding the circuit in a structural thermoplastic encasement;
Figure 3 illustrates a method of manufacturing the tape assembly in accordance with a first exemplary aspect;
Figure 4 illustrates a method of manufacturing the tape assembly in accordance with a second exemplary aspect;
Figure 5 illustrates a method of applying the tape assembly to the catheter shaft via a heat transfer process;
Figure 6A is a side view of the tape assembly constructed in accordance with a third embodiment and illustrating the circuit assembly including a plurality of conductive circuit layers and a plurality of dielectric layers disposed in alternating stacked relationship with one another;
Figure 6B is a side view of the tape assembly illustrating an alternative arrangement of the third embodiment that incorporates more complex circuit patterns in place of the contact pads in the circuit to incorporate further functionality into the circuit assembly;
Figures 7A is a top view of the tape assembly in accordance with any of the aforementioned embodiments to illustrate a different arrangement of the conductive circuit layer of the circuit assembly including a plurality of contact pads disposed at an end of the circuit;
Figure 7B is a top view of the tape assembly in accordance with any of the aforementioned embodiments to illustrate a different arrangement of the conductive circuit layer of the circuit assembly in which sensors replace the contact pads of the circuit; and
Figure 7C is a top view of the tape assembly in accordance with any of the aforementioned embodiments to illustrate a different arrangement of the conductive circuit layer of the circuit assembly in which round wires can be incorporated into the circuit as a substitute for a flat conductive metal.

### DESCRIPTION OF THE ENABLING EMBODIMENTS

Example embodiments will now be described more fully with reference to the accompanying drawings. The example embodiments are provided so that this disclosure will be thorough and fully convey the scope to those skilled in the art. Numerous specific details are set forth such as examples of specific components, devices, mechanisms, assemblies and methods to provide a thorough understanding of various embodiments of the present disclosure. It will be apparent to those skilled in the art that specific details need not be employed, that example embodiments may be embodied in many different forms, and that neither should be construed to limit the scope of the disclosure. With this in mind, the present disclosure is generally directed to a tape assembly **10** for applying a circuit **20** to a catheter shaft **30** during manufacture of a smart catheter.

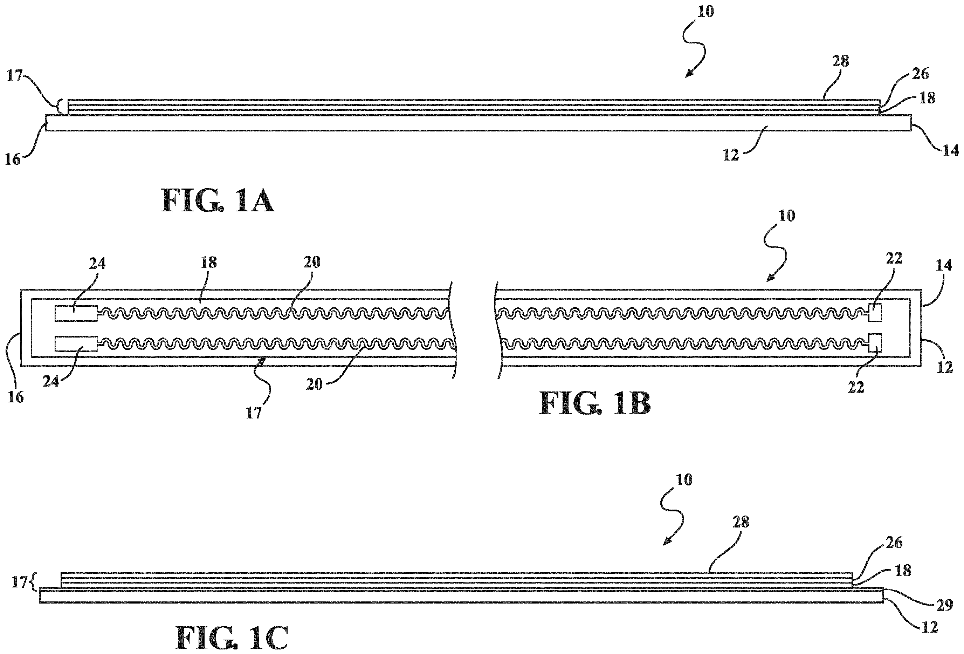

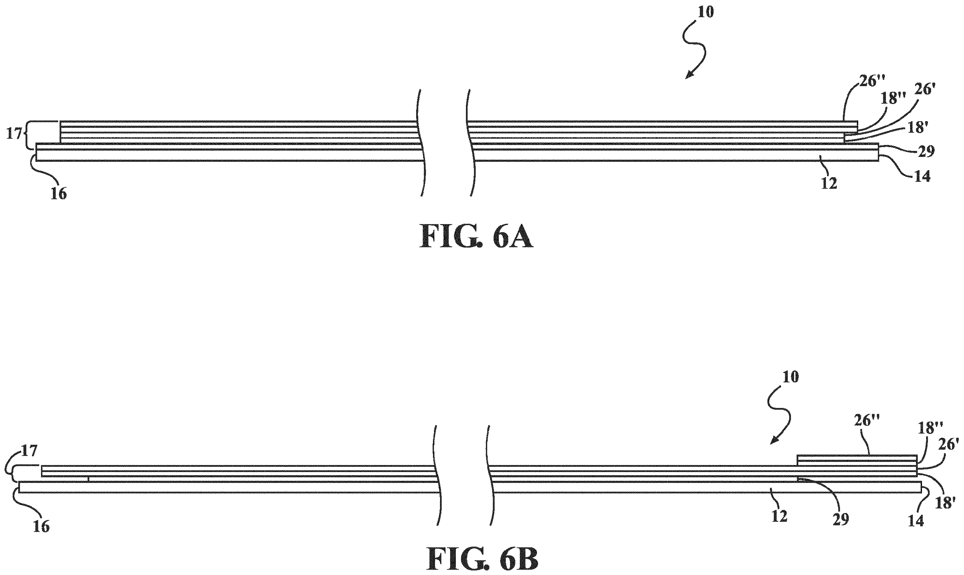

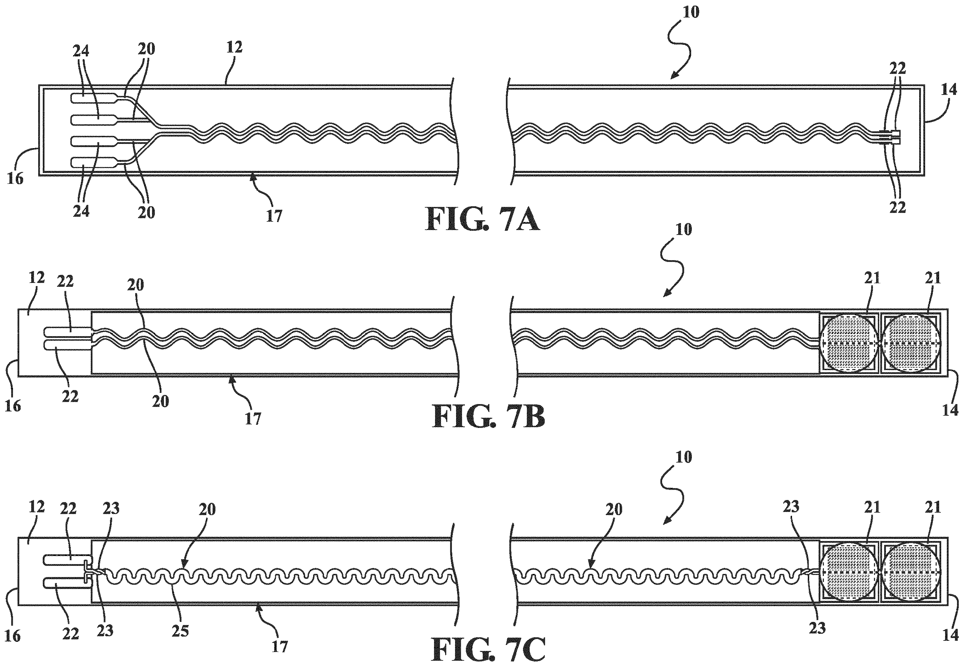

Referring to the Figures, wherein like numerals indicate corresponding parts throughout the several views, the tape assembly **10** is generally shown in Figures 1A, 1C and 6A-6B. As best illustrated in Figure 1A, 1C and 6A-6B, the tape assembly **10** includes a removable transfer media layer **12** extending between a first end **14** and a second end **16,** and a circuit assembly **17** overlaying and removably bonded to the removable transfer media layer **12** between the first and second ends **14, 16.** The circuit assembly **17** includes a conductive circuit layer **18** having at least one circuit **20.** As best illustrated in Figure 1B in accordance with an aspect, the at least one circuit **20** of the circuit assembly **17** extends between a first contact pad **22** disposed adjacent the first end **14** of the removable transfer media layer **12** and a second contact pad **24** disposed adjacent the second end **16** of the removable transfer media layer **12.** As best illustrated in Figure 7A, each circuit **30** can include a plurality of contact pads **22, 24** at one of (or both) of the ends **14, 16.** Furthermore, as illustrated in Figure 7C, the circuit **20** can also include at least one sensor **21,** or other electrical and electronic components, at one or both of the ends **14, 16** in lieu of the contact pads **22, 24** to incorporate further functionality into the circuit assembly. The at least one circuit **20** is preferably comprised of conductive metal materials, such as copper due to its low cost and high conductivity, but other conductive metal materials such as gold or silver could be utilized without departing from the scope of the subject disclosure. Furthermore, as illustrated in Figure 7C, the at least one circuit **20** can include a plurality of round twisted wires **23** extending between the contact pads **22, 24** or sensors **21** and encased within a sheath **25** (such as to eliminate or reduce noise generated from the circuit **20**) in lieu of the flat conductive metal shown in Figures 1A and 7A-7B.

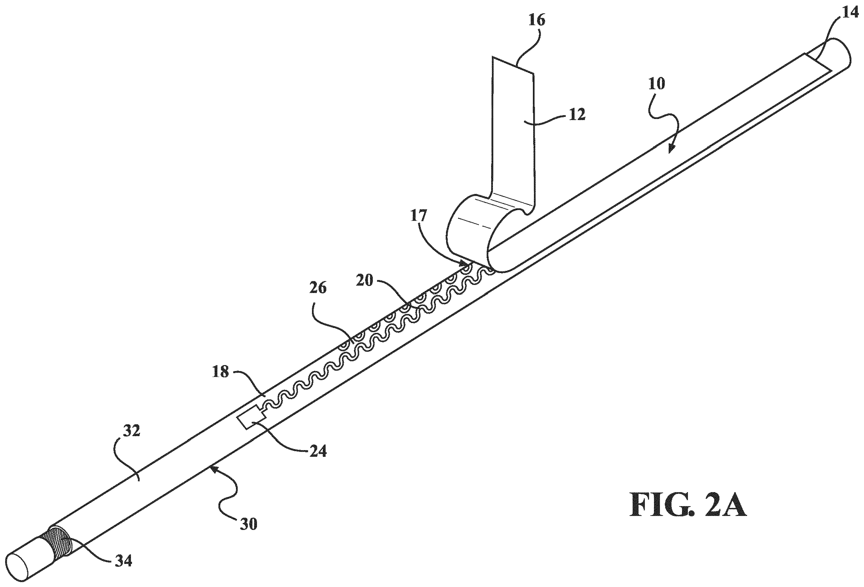

The removable transfer media layer **12** can be comprised of any media capable of transferring the circuit assembly **17** to another substrate, such as an outer surface **32** of a catheter shaft **30** as shown in Figure 2A, without deforming or damaging the circuit assembly **17.** In other words, the removable transfer media layer **12** is comprised of a material strong enough to support and maintain an integrity and structure of the thin, circuit assembly **17** including the conductive circuit layer **18** (e.g. a layer that can be on the micron scale) until a transfer to the catheter shaft **30** is made. For example, as will be described in the following more detailed disclosure, the removable transfer media layer **12** in accordance with an aspect can be comprised of a heat transfer tape or heat release tape, each of which releases the circuit assembly **17** from the transferable media layer **12** when a temperature is obtained for a predetermined amount of time. However, the transferable media layer **12** can include other types of releasable media such as heat resistant or other media that does not require application of heat to effectuate a transfer, such as low adhesion tape, without departing from the subject disclosure.

In any arrangement, the removable transfer media layer **12** provides temporary structural support for the circuit assembly **17** until transferred to become part of the catheter shaft **30.** In other words, the removable transfer media layer provides structural support before and during the transfer of the circuit assembly **17.** As will be explained in more detail below, once the transfer is complete, the removable transfer media layer **12** can be removed and discarded leaving only the circuit assembly **17** behind. And since the circuit assembly **17** does not need to provide structure support on its own, much of the materials used to build the circuit assembly **17** can be eliminated or ultra-thin, measuring only microns, resulting in a smart catheter with a circuit **20** having a smaller diameter relative to the prior art (*See* Figure 2C compared to Figure 2D).

As further illustrated in Figures 1A, 1C and 6A-6B, the circuit assembly **17** is preferably multi-layered and additionally includes a dielectric layer **26** disposed in overlaying relationship with the conductive circuit layer **18** to dispose the conductive circuit layer **18** in sandwiched relationship between the dielectric layer **26** and the removable transfer media layer **12.** However, in an aspect, the circuit assembly **17** could only be comprised of the conductive circuit layer **18** without departing from the scope of the subject disclosure. The dielectric layer **26,** if present, protects the conductive circuit layer **18** if a risk of shorting is a concern, such as when a braid layer **34** is present on the catheter shaft **30** (as shown in Figures 2A-B). As illustrated in Figure 2A, when the tape assembly **10** (and thus the circuit assembly **17**) is applied to the catheter shaft **30,** the dielectric layer **26** is disposed in overlaying relationship with the conductive circuit layer **18** so that it is facing downwards towards the outer surface **32,** and particularly the braid layer **34,** of the catheter shaft **30.** Thus the dielectric layer **26** helps to ensure that the conductive circuit layer **18** does not contact the exposed braid **34** of the catheter shaft **30** which lies radially inward from and just beneath or along the outer surface **32.** In other words, the dielectric layer **26** is disposed fully or in part onto the conductive circuit layer **18** to avoid short circuits, such as when multiple conductor circuit layers are stacked on top of one another *(See* Figures 6A-6B) or when the conductive circuit layer **18** could be unintentionally exposed to other conductive materials, such as the braid layer **34.** As illustrated in Figures 6A-6B, when a plurality of conductor circuit layers **18', 18"** are present, a plurality of dielectric layer **26', 26"** are also utilized and can be disposed between each adjacent conductive circuit layer **18', 18"** to dispose multiple conductive circuit layers **18', 18"** and multiple dielectric layers in stacked relationship with one another **26', 26".** For example, a first dielectric layer **26'** can be disposed in facing relationship with the outer surface **32** of the catheter shaft **30,** followed by a first conductive circuit layer **18',** then a second dielectric layer **26",** and then a second conductive circuit layer **18",** etc. In any arrangement - with single layers or multiple layers, the dielectric layer **26** makes the overall device more robust due to the reduced likelihood of circuit breakage or shorting. However, if the catheter shaft **30** does not have a risk of shorting, the dielectric layer **26** could be omitted without departing from the scope of the subject disclosure.

As illustrated in Figures 1A and 1C, the circuit assembly **17** can additionally include an adhesive layer **28** disposed in overlaying relationship with the dielectric layer **26** for use in securing the circuit assembly **17** to the outer surface **32** of the catheter shaft **30.** In other words, the adhesive layer **28** assists in bonding the circuit assembly **17** to the catheter shaft **30,** or directly to the braid layer **34.** Thus, the adhesive layer **28** is placed on the outer most surface of the circuit assembly **17,** farthest away from the removable transfer media layer **12.** However, in accordance with certain aspects, while the adhesive layer **28** can assist to effectuate transfer and bonding of the circuit assembly **17,** the adhesive layer **28** could be removed especially if the circuit assembly **17** is heat transferred or reflowed on the catheter shaft **30,** such as will be explained in more detail below. In this situation, with enough heat the circuit assembly **17** will bond mechanically to the thermoplastic catheter shaft **30** as the conductive circuit layer **18** is pushed down into the melted plastic. Thus, in accordance with the subject disclosure, the thermoplastic on the catheter shaft **30** can be used to bond the circuit assembly **17** in lieu of the adhesive layer **28.**

As best illustrated in Figure 2A, in accordance with an aspect, the conductive circuit layer **18** is transferred to the catheter shaft **30** by applying the tape assembly **10** to the outer surface **32** of the catheter shaft **30,** disposing the adhesive layer **28** (if present) and the dielectric layer **26** (if present) facing down and sequentially overlaying the outer surface **32** of the catheter shaft **30.** Once applied, the transferable media layer **12** is removed from the ta tape assembly **10,** leaving the circuit assembly **17** secured to the outer surface **32** of the catheter shaft **30,** via either the additional adhesive layer **28** or with the other heat transferring or reflowing procedures mentioned above (if adhesive is not present). As illustrated in Figure 2A, after application of the tape assembly **10,** the removable transfer media layer **12** can be peeled away from the circuit assembly **17** and discarded.

As described above, when the tape assembly **10,** including the removable transfer media layer **12** and the circuit assembly **17,** is applied to the catheter shaft **30,** the dielectric layer **26** faces down and overlays the outer surface **32** of the catheter shaft **30.** Then, the removable transfer media layer **12** is removed from the circuit assembly **17,** which is now part of the construction of the catheter shaft **30,** leaving the conductive circuit layer **18** potentially exposed to an environment of the catheter shaft **30.** Thus, as illustrated in Figures 1C and 6A-6B, in an accordance with a second embodiment of the subject disclosure, the circuit assembly **17** can additionally include a seal layer **29** that is disposed in sandwiched relationship between the transferable media layer **12** and the conductive circuit layer **18.** When the removable transfer media layer **18** is removed from the circuit assembly **17,** the seal layer **29** overlays and protects the conductive circuit layer **18** from the environment (e.g., the human body), providing an efficient way to protect the conductive circuit layer **12** without the need to incorporate additional and costly process steps at a later time to provide this same amount of protection for the conductive circuit layer **18.** In a preferred arrangement, the seal layer **29** is a very thin layer of polymer material applied to the conductive circuit layer **18** (such as via lamination, coating, spraying, plating processes, or the like). Accordingly, as best illustrated in Figures 2C, this very thin seal layer **29** has a minimal effect on the overall size of the catheter shaft **30,** yet still provides protective benefits for the conductive circuit layer **18** without requiring the need to incorporate additional process steps later when manufacturing the catheter shaft **30** to provide this same level of protection. Thus, these reduced processing steps for the catheter shaft **30** lead to lower manufacturing and product costs. While the seal layer **29** is described preferably as a polymer, the seal layer **29** could be any other bio-compatible flexible material without departing from the scope of the subject disclosure.

The tape assembly **10,** including the removable transfer media layer **12** and the circuit assembly **17,** provides a number of advantages over the prior art methods of incorporating a circuit into the catheter shaft **30** to manufacture a smart catheter. Initially, the removable (and disposable) transferable media layer **12** prevents additional thickness being added to the catheter shaft **30** by way of the application of circuit assembly **17** (since the transfer media layer **12** can be removed after application of the circuit assembly **17** to the catheter shaft **30**). This allows for a significant decrease in the overall catheter size (i.e., diameter), which is crucial to minimizing trauma of the patient when the catheter shaft **30** is ultimately in use during a medical procedure. Put another way, the overall thickness of the circuit assembly **17** applied to the catheter shaft **30** (after the transferable media layer **12** is removed) is very thin, providing a smart circuit on the catheter shaft **30,** achieving a smaller profile than achievable by the prior art methods of incorporating the circuits via electrical wiring or reflowing the circuit embedded in a structural thermoplastic encasement **36,** such as shown in Figure 2C (subject arrangement) compared to Figure 2D (prior art).

Furthermore, application of the circuit assembly **17** to an outer surface **32** of the catheter shaft **30** provides a method of manufacturing a smart catheter with reduced cost, reduced assembly time and increased yields over the prior art manufacturing methods. For example, the ability to transfer the circuit assembly **17** directly onto the catheter shaft **30** eliminates the requirement to have the circuit in a structural thermoplastic encasement **36** prior to transfer of the circuit **20** to the catheter shaft (such as required in the prior art illustrated in Figure 2D), and keeps the circuit **20** stable with minimal chance of movement during transfer to the catheter shaft **30.** After transfer of the conductive circuit layer **18** to the catheter shaft **30,** the catheter shaft **30** can then move on to other processes required to complete the catheter assembly. For example, it is likely that the contact pads **22, 24** or sensors **21** will need to be exposed, while at the same time the circuit **20** between the contact pads **22, 24** or sensors **21** will need to be covered. One way to accomplish this is by masking what you don't want covered, such as the contact pads **22, 24** or sensors **21,** prior to coating the catheter shaft **30** to seal in the areas of the circuit **20** between the contact pads **22, 24** or sensors **21.** Methods of coating could be but are not limited to dip, spray and vapor deposition to just name a few. Another possible way to cover the conductive circuit layer **18** is by applying another thin layer of thermoplastic material over the areas that need to be protected against the environment. In cases when the circuit **20** is bonded directly to the braid layer **34,** a reflow process can be utilized in which a non-conductive material such as thermoplastic is applied over the braid layer **34** and the circuit **20** which is affixed to the braid layer **34.**

Inclusion of the dielectric layer **26** in the circuit assembly **17** prior to transfer of the at least one conductive circuit layer **18** onto the catheter shaft **30** also helps to ensure that the conductive circuit layer **18** will not short out and be damaged during manufacture, such as by contacting the braid layer **34** in the catheter shaft **30.** Thus, the dielectric layer **26** allows the conductive circuit layer **18** to be applied to a wide range of outer surfaces, including coils or braid layers. Additionally, use of the removable transfer media layer **12** in the tape assembly **10** keeps the conductive circuit layer **18** stable during transfer to the catheter shaft **30,** preventing the conductive circuit layer **18** from floating and making the overall device more robust due to the reduced likelihood of circuit shorting, as well as the elimination of small, fragile wires which are prone to breakage in the prior art designs. Further, presence of the seal layer **29** makes a bond between the transferable media layer **12** and the conductive circuit layer **18** less critical, namely because it eliminates any gaps between these two surfaces which could cause failures in prior art devices while etching in an etchant bath or spray during manufacturing of the circuit assembly. Finally, the tape assembly **10** provides for increased opportunity for attaching customized electrodes or sensors **21** when manufacturing the smart catheter as illustrated in Figures 7A-7C.

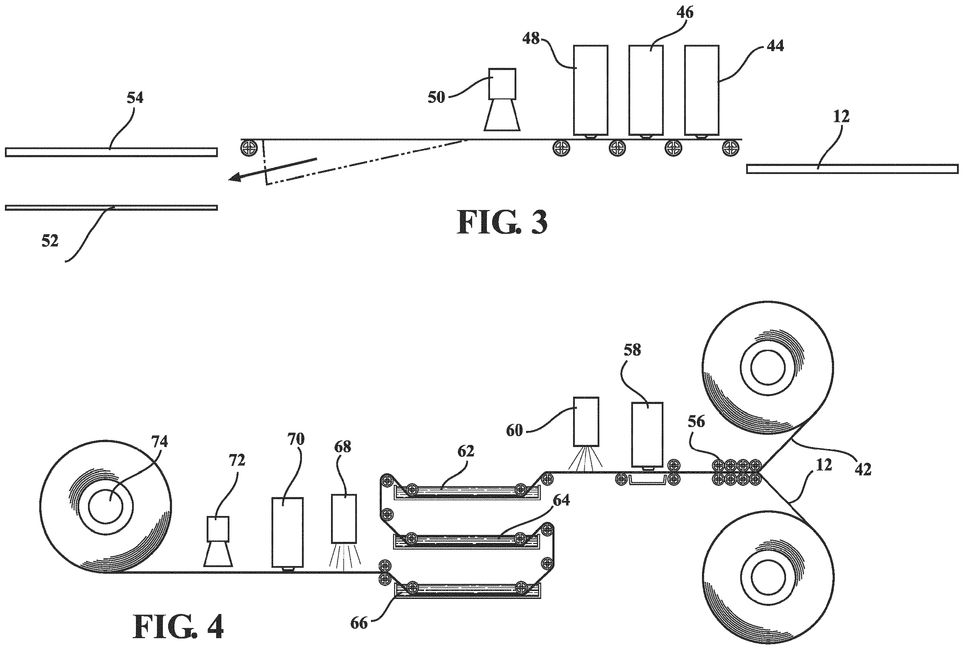

As mentioned previously, the conductive circuit layer **18** is comprised of any electronic and/or electrical components used to establish the circuit **20**, and could range anywhere from conductive inks to electroplating and chemical milling (also referred to as chemical etching). In certain instances, as illustrated in Figure 7C, a plurality of twisted wires **23** encased within a sheath **25** may also be a desired approach for the circuit **20**, due to the noise-cancelling and shielding effects provided by the sheath **25.** Conductive inks might also be the desired approach to manufacture the conductive circuit layer **18** due to their manufacturing simplicity. If conductive inks are used, there are many ways to apply the conductive ink circuit to the transferable media layer **12,** such as via screen printing, inkjet, extrusion printing, aerosol jet, electroplating, laser sintering, pulse light sintering, and the like. Figure 3 illustrates an exemplary method of manufacturing the tape assembly **10** via a conductive ink approach, in which the process begins with pre-bounded sheets of a predetermined length consisting of the removable transfer media layer **12.** Each sheet is sequentially passed through a conductive ink print station **44** for applying the conductive circuit layer **18,** a dielectric ink print station **46** for applying the dielectric layer **26,** and an adhesive ink print station **48** for applying the adhesive layer **28** and completing manufacture of the tape assembly **10.** Due to the nature of working with individual pieces, the manufactured sheet of the tape assembly **10** can then be passed through an inspection station **50** to identify rejected product and good product, after which the rejected product is passed to a reject stack **52** and good product is passed to a circuit stack **54.**

While manufacturing the conductive circuit layer **18** via conductive inks is suitable, the preferred method of manufacturing is via chemical etching due to the cost, strength characteristics and superior conductivity of 99% copper. The use of chemical etching to manufacture the conductive circuit layer **18** achieves the conductivity needs of conductive circuit layer **18** in the circuit assembly **17** at very thin thicknesses. These thin layers for the conductive circuit layer **18** help to keep an outer diameter of the resultant smart, catheter shaft **30** at a minimum, thus reducing trauma to the patient during a procedure.

There are a variety of ways to approach copper etching, with use of a photo-resist film or photo-resist coating being a commonly used process. However, with reference to Figure 4, a preferred approach of chemical etching to manufacture the conductive circuit layer **18** is via use of a UV curable dielectric ink. This manufacturing process allows a number of the requisite process steps with use of photo-resist film processing to be eliminated. Additionally, because the circuit assembly **17** on the tape assembly **10** includes a dielectric layer **26,** there is no need to remove this dielectric layer **26** after etching has been completed.

With reference to Figure 4, in accordance with an exemplary aspect, the UV curable dielectric ink manufacturing process begins with the removable transfer media layer **12** being pressed and bound together with clean copper foil **42** using pressure rollers **56.** The combined transfer media layer **12** and copper foil **42** then passes through a dielectric ink print station **58** where a desired circuit is printed on the copper foil **42** using UV curable dielectric ink. The printed assembly then passes through a UV curing station **60** followed by an etching cycle sequentially comprised of an etching bath or spray **62,** an etchant neutralizing bath or spray **64** and a rinse bath or spray **66** to form the conductive circuit layer **18.** When the etched conductive circuit layer **18** comes out of the rinse bath or spray **66,** it is then dried in a dry station **68,** after which the optional adhesive layer **28** can be applied via an adhesive dispenser **70.** The completed tape assembly **10** then passes through an inspection camera **72** after which it is rolled onto a spool **74** and ready for use and application to a catheter shaft **30.**

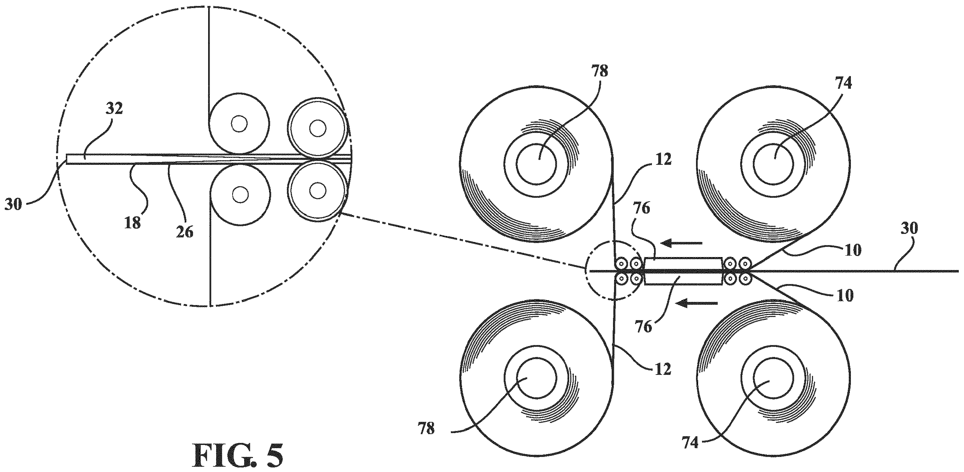

Once the tape assembly **10** is manufactured and spooled onto a continuous roll, it can then be transferred to the catheter shaft **30** via several ways. As mentioned previously, a preferred method of transferring the circuit assembly **17** to the catheter shaft **30** is via a heat transfer process, such as shown in Figure 5. As an example of this process, the catheter shaft **30** is inserted into and passed along a conveyor system, while at least one spool **74** of the tape assembly **10** is pressed against the catheter shaft **30** as the catheter shaft **30** advances through a heat station **76.** If the adhesive layer **28** is not present, the heat helps to mechanically bond the conductive material of the conductive circuit layer **18** to the thermoplastic material of the catheter shaft **30,** while at the same time lowering a force of an adhesive bond between the removable transfer media layer **12** and the conductive circuit layer **18.** In other words, heat has two primary functions during transfer of the circuit assembly **17** to the catheter shaft **30.** First, the heat lessens the holding force of the conductive circuit layer **18** from the removable transfer media layer **12.** Second, if the conductive circuit layer **18** is applied directly to the thermoplastic polymer of the catheter shaft **30,** the heat helps to mechanically lock the circuit assembly **17** securely to the catheter shaft **30.** After passing through the heating station **76,** the removable transfer media layer **12** is then lifted off of the circuit assembly **17** leaving the circuit assembly **17** behind. The removable transfer media layer **12** can be gathered in a release spool **78.** If present, the seal layer **29** could make releasing the circuit assembly **17** from the transferable media layer **12** easier since bonding to high energy surfaces such as PeBax creates a lesser bond than when bonded directly to copper, such as present in the preferred composition of the conductive circuit layer **18.**

Although described and illustrated as being applied to the outer surface **32** of the catheter shaft **30,** inclusion of the adhesive layer **28** allows the circuit assembly **17** to be applied earlier in the smart catheter manufacturing process, such as being applied directly to the braid layer **34,** prior to the braid layer **34** being covered with polymer to form the outer surface **32.**

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A tape assembly for transferring at least one circuit onto a catheter shaft, the tape assembly comprising:
a removable transfer media layer extending between a first end and a second end;
a circuit assembly disposed in overlaying and releasably bonded relationship with said removable transfer media layer between said first and second ends;
said circuit assembly including a conductive circuit layer having at least one circuit;
wherein said removable transfer media layer is removable from said circuit assembly after being transferred to the catheter shaft.

2. The tape assembly as set forth in Claim 1, wherein said circuit assembly includes a dielectric layer disposed in overlaying relationship with said conductive circuit layer to dispose said conductive circuit layer in sandwiched relationship between said dielectric layer and said removable transfer media layer.

3. The tape assembly as set forth in Claim 1, wherein said circuit assembly includes an adhesive layer disposed in overlaying relationship with said dielectric layer for use in securing said circuit assembly to the catheter shaft.

4. The tape assembly as set forth in Claim 1, wherein said circuit assembly includes a seal layer disposed in sandwiched relationship between said transfer media layer and said conductive circuit layer for protecting said conductive circuit layer from an environment of the catheter shaft after removal of said removable transfer media layer.

5. The tape and transferable circuit assembly as set forth in Claim 1, wherein said transferable media layer is a heat transfer tape, a heat release tape or a low adhesion tape.

6. The tape assembly as set forth in Claim 1, wherein said at least one circuit extends between at least one first contact pad disposed adjacent said first end of said removable transfer media layer and at least one second contact pad disposed adjacent said second end of said removable transfer media layer.

7. The tape assembly as set forth in Claim 1, wherein said conductive circuit layer and said at least one circuit are comprised of conductive material.

8. The tape assembly as set forth in Claim 1, wherein said conductive circuit layer is manufactured via conductive inks or chemical etching.

9. The tape assembly as set forth in Claim 1, wherein said circuit assembly is transferred to the catheter shaft via a heat transfer process.

10. A method of manufacturing a smart catheter comprising:
providing a catheter shaft
providing a tape assembly including a removable transfer media layer and a circuit assembly having a conductive circuit layer;
applying the tape assembly to the catheter shaft to dispose the conductive circuit layer in overlaying relationship with the catheter shaft; and
removing the transferable media layer from the conductive circuit layer to leave the conductive circuit layer applied and transferred to the catheter shaft.

11. The method of manufacturing the smart catheter as set forth in Claim 10, wherein the circuit assembly additionally includes a dielectric coating layer disposed in overlaying relationship with the conductive circuit layer, and wherein said step of applying the tape assembly includes disposes the dielectric coating layer in overlaying relationship with the catheter shaft to isolate the conductive circuit layer from an outer surface or a braid layer of the catheter shaft.

12. The method of manufacturing the smart catheter as set forth in Claim 10, wherein the circuit assembly additionally includes an adhesive layer disposed in overlaying relationship with the dielectric coating for use in securing the circuit assembly to the catheter shaft.

13. The method of manufacturing the smart catheter as set forth in Claim 10, wherein the circuit assembly additionally includes a seal layer disposed in sandwiched relationship between the transferable media layer and the conductive circuit layer, and said step of removing the removable transfer media layer includes leaving the seal layer in overlaying relationship with the conductive circuit layer to protect the conductive circuit layer from an environment of the catheter shaft.

14. The method of manufacturing the smart catheter as set forth in Claim 10, wherein the removable transfer media layer is comprised of a heat transfer tape or heat release tape, and said step of transferring the tape assembly to the catheter shaft includes a heat transfer process.

15. The method of manufacturing the smart catheter as set forth in Claim 10, wherein the conductive circuit layer includes at least one circuit and is manufactured via conductive inks or chemical etching.
